# EUROPEAN PATENT APPLICATION

(11) **EP 1 258 530 A2**
(43) Date of publication of application: **20.11.2002**
(21) Application number: 02078136.5
(22) Date of filing: 09.06.1992
(51) Int. Cl.: C12N 15/12, C12N 5/10, C07K 14/705, G01N 33/566

(54) **Gaba-A receptor subunits (alpha-2, alpha-3, alpha-5, alpha -6, beta-2) and transfected cells expressing them**

(30) Priority: 11.06.1991 GB 9112504
(62) Divisional of application: 92911528.5
(71) Applicant: MERCK SHARP & DOHME LTD., Hoddesdon Hertfordshire EN11 9NU (GB)
(72) Inventor: Handingham, Karen Louise, Harlow, Essex CM20 2QR (GB); Le Bourdelles, Beatrice, Harlow, Essex CM20 2QR (GB); Whiting, Paul John, Harlow, Essex CM20 2QR (GB); Wingrove, Peter Baxter, Harlow, Essex CM20 2QR (GB)
(74) Representative: Horgan, James Michael Frederic

(57) **Abstract**

The present invention relates to a stably co-transfected eukaryotic cell line capable of expressing a GABA_{A} receptor, particularly a human GABA_{A} receptor, which receptor comprises at least one alpha, one beta and one gamma subunit; to the cloning of novel cDNA sequences encoding the (α-2, α-3, α-5, α-6 and β-2 subunits of the human GABA_{A} receptor; and to the use of the cell line in designing and developing GABA_{A} receptor subunit-selective medicaments.

## Description

This invention concerns a cell line, and in particular relates to a stable cell line capable of expressing human or animal GABA_{A} receptors. The invention further concerns the cloning of novel cDNA sequences encoding particular subunits of the human GABA_{A} receptor. In addition, the invention relates to the use of the cell line in a screening technique for the design and development of subtype-specific medicaments.

Gamma-amino butyric acid (GABA) is a major inhibitory neurotransmitter in the central nervous system. It mediates fast synaptic inhibition by opening the chloride channel intrinsic to the GABA_{A} receptor. This receptor comprises a multimeric protein of molecular size 230-270 kDa with specific binding sites for a variety of drugs including benzodiazepines, barbiturates and β-carbolines, in addition to sites for the agonist ligand GABA (for reviews see Stephenson, Biochem. J., 1988, 249, 21; Olsen and Tobin, Faseb J., 1990, 4, 1469; and Sieghart, Trends in Pharmacol. Sci., 1989, 10, 407).

Molecular biological studies demonstrate that the receptor is composed of several distinct types of subunit, which are divided into four classes (α, β, γ, and δ) based on their sequence similarities. To date, six types of α (Schofield et al., Nature (London), 1987, 328, 221; Levitan et al., Nature (London), 1988, 335, 76; Ymer et al., EMBO J., 1989, 8, 1665; Pritchett & Seeberg, J. Neurochem., 1990, 54, 802; Luddens et al., Nature (London), 1990, 346, 648; and Khrestchatisky et al., Neuron, 1989, 3, 745), three types of β (Ymer et al., EMBO J., 1989, 8, 1665), two types of γ (Ymer et al., EMBO J., 1990, 9, 3261; and Shivers et al., Neuron, 1989, 3, 327) and one δ subunit (Shivers et al., Neuron, 1989, 3, 327) have been identified.

The differential distribution of many of the subunits has been characterised by in situ hybridisation (Sequier et al., Proc. Natl. Acad. Sci. USA, 1988, 85, 7815; Malherbe et al., J. Neurosci., 1990, 10, 2330; and Shivers et al., Neuron, 1989, 3, 327) and this has permitted it to be speculated which subunits, by their co-localisation, could theoretically exist in the same receptor complex.

Various combinations of subunits have been co-transfected into cells to identify synthetic combinations of subunits whose pharmacology parallels that of bona fide GABA_{A} receptors in vivo (Pritchett et al., Science, 1989, 245, 1389; Malherbe et al., J. Neurosci., 1990, 10, 2330; Pritchett and Seeberg, J. Neurochem., 1990, 54, 1802; and Luddens et al., Nature (London), 1990, 346, 648). This approach has revealed that, in addition to an α and β subunit, either γ₁ or γ₂ (Pritchett et al., Nature (London), 1989, 338, 582; Ymer et al., EMBO J., 1990, 9, 3261; and Malherbe et al., J. Neurosci., 1990, 10, 2330) or γ₃ (Herb et al., Proc. Natl. Acad. Sci. USA, 1992, 89, 1433; Knoflach et al., FEBS Lett., 1991, 293, 191; and Wilson-Shaw et al., FEBS Lett., 1991, 284, 211) is also generally required to confer benzodiazepine sensitivity, and that the benzodiazepine pharmacology of the expressed receptor is largely dependent on the identity of the α and γ subunits present. Receptors containing a δ subunit (i.e. αβδ) do not appear to bind benzodiazepines (Shivers et al., Neuron, 1989, 3, 327). Combinations of subunits have been identified which exhibit the pharmacological profile of a BZ₁ type receptor (α₁β₁γ₂) and a BZ₂ type receptor (α₂β₁γ₂ or α₃β₁γ₂, Pritchett et al., Nature (London), 1989, 338, 582), as well as two GABA_{A} receptors with a novel pharmacology, α₅β₂γ₂ (Pritchett and Seeberg, J. Neurochem., 1990, 54, 1802) and α₆β₂γ₂ (Luddens et al., Nature (London), 1990, 346, 648). Although the pharmacology of these expressed receptors appears similar to that of those identified in brain tissue by radioligand binding, it has nonetheless not been shown that these receptor subunit combinations exist in vivo.

The present invention is concerned with the production of permanently transfected cells containing the GABA_{A} receptor, which will be useful for screening for drugs which act on this receptor. The GABA_{A} receptor has previously been expressed in Xenopus oocytes (Sigel et al., Neuron, 1990, 5, 703-711) and in transiently transfected mammalian cells (Pritchett et al., Science, 1989, 245, 1389-1392). However, both of those systems involve transient expression and are unsuitable for screening purposes.

We have now achieved the stable expression of the receptor.

Accordingly, the present invention provides a stably co-transfected eukaryotic cell line capable of expressing a GABA_{A} receptor, which receptor comprises at least one alpha, one beta and one gamma subunit.

This has been achieved by co-transfecting cells with three expression vectors, each harbouring cDNAs encoding for an α, β or γ GABA_{A} receptor subunit. In a further aspect, therefore, the present invention provides a process for the preparation of a eukaryotic cell line capable of expressing a GABA_{A} receptor, which comprises stably co-transfecting a eukaryotic host cell with at least three expression vectors, one such vector harbouring the cDNA sequence encoding for an alpha, another such vector harbouring the cDNA sequence encoding for a beta, and a third such vector harbouring the cDNA sequence encoding for a gamma GABA_{A} receptor subunit. The stable cell-line which is established expresses an αβγ GABA_{A} receptor. Each receptor thereby expressed, comprising a unique combination of α, β and γ subunits, will be referred to hereinafter as a GABA_{A} receptor "subunit combination". Pharmacological and electrophysiological data confirm that the recombinant αβγ receptor expressed by the cells of the present invention has the properties expected of a native GABA_{A} receptor.

Expression of the GABA_{A} receptor may be accomplished by a variety of different promoter-expression systems in a variety of different host cells. The eukaryotic host cells suitably include yeast, insect and mammalian cells. Preferably the eukaryotic cells which can provide the host for the expression of the receptor are mammalian cells. Suitable host cells include rodent fibroblast lines, for example mouse Ltk⁻, Chinese hamster ovary (CHO) and baby hamster kidney (BHK); HeLa; and HEK293 cells. It is necessary to incorporate at least one α, one β and one γ subunit into the cell line in order to produce the required receptor. Within this limitation, the choice of receptor subunit combination is made according to the type of activity or selectivity which is being screened for. For example, benzodiazepines (designated BZ) represent one class of drugs which act upon the GABA_{A} receptor. The presence of an α₁ subunit is specific for a class of benzodiazepines having the pharmacology designated BZ₁; whereas α₂ to α₅ define different pharmacological profiles, broadly designated as BZ₂. The type of β subunit is not critical in defining the class of benzodiazepine, although a β subunit is required. The γ subunit is also important in defining BZ selectivity. It is likely that differentiation between α subunit selectivity is conferred by the identity of the particular γ subunit present.

In order to employ this invention most effectively for screening purposes, it is preferable to build up a library of cell lines, each with a different combination of subunits. Typically a library of 5 or 6 cell line types is convenient for this purpose. Preferred subunit combinations include: α₁β₁γ₂; α₁β₂γ₂; α₂β₁γ₁; α₂β₁γ₂; α₂β₁γ₃; α₃β₁γ₂; α₃β₁γ₃; α₄β₁γ₂; α₅β₁γ₂; and α₆β₁γ₂; especially α₁β₁γ_{2L}.

The DNAs for the receptor subunits can be obtained from known sources, and are generally obtained as specific nucleotide sequences harboured by a standard cloning vector such as those described, for example, by Maniatis et al. in Molecular Cloning, A Laboratory Manual, Cold Spring Harbor Press, New York, 2nd edition, 1989. Preferably the cDNA sequences are derived from the human gene. However, for screening purposes, cDNAs from other species are also suitable, such as bovine or rat DNA. Known sources of GABA_{A} receptor subunit cDNAs are as follows:

α1 bovine )    Schofield et al., Nature, 1987, 328,
β1 bovine )    221-227.

α1 human )    Schofield et al., FEBS Lett., 1989, 244,
β1 human )    361-364.

α2 rat    Khrestchatisky et al., J. Neurochem.,
1991, 56, 1717.

α2 bovine ) Levitan et al., Nature, 1988, 335,
α3 bovine ) 76-79.

α4 rat Wisden et al., FEBS Lett., 1991, 289, 227.

α4 bovine Ymer et al., FEBS Lett., 1989, 258,
119-122.

α5 rat Pritchett and Seeburg,
J. Neurochem., 1990, 54, 1802-1804.

α6 rat ) Luddens et al., Nature, 1990, 346,
α6 bovine ) 648-651.

β2 bovine ) Ymer et al., EMBO J., 1989, 8, 1665-1670.
β2 rat )
β3 bovine )
β3 rat )

γ1 human ) Ymer et al., EMBO J., 1990, 9, 3261-3267.
γ1 rat )
γ1 bovine )

γ2 human Pritchett et al., Nature, 1989, 338,
582-585.

γ2 bovine Whiting et al., Proc. Natl. Acad.
Sci. USA, 1990, 57, 9966-9970.

γ3 rat Herb et al., Proc. Natl. Acad. Sci. USA,
1992, 89, 1433; and
Knoflach et al., FEBS Lett., 1991, 293,
191.

γ3 mouse    Wilson-Shaw et al., FEBS Lett., 1991, 284,
211.

δ rat    Shivers et al., Neuron, 1989, 3, 327.

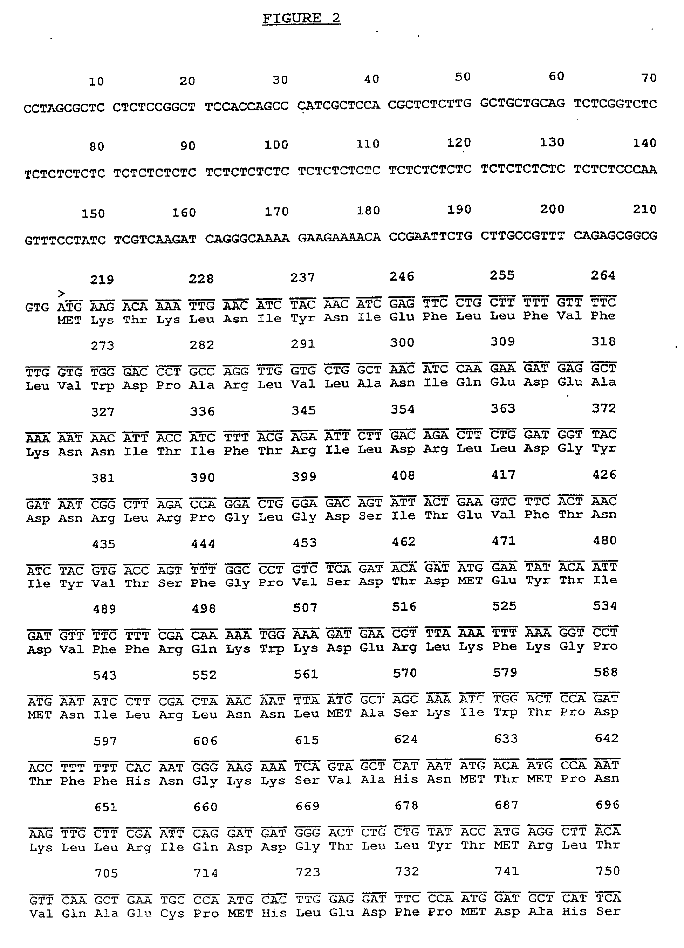

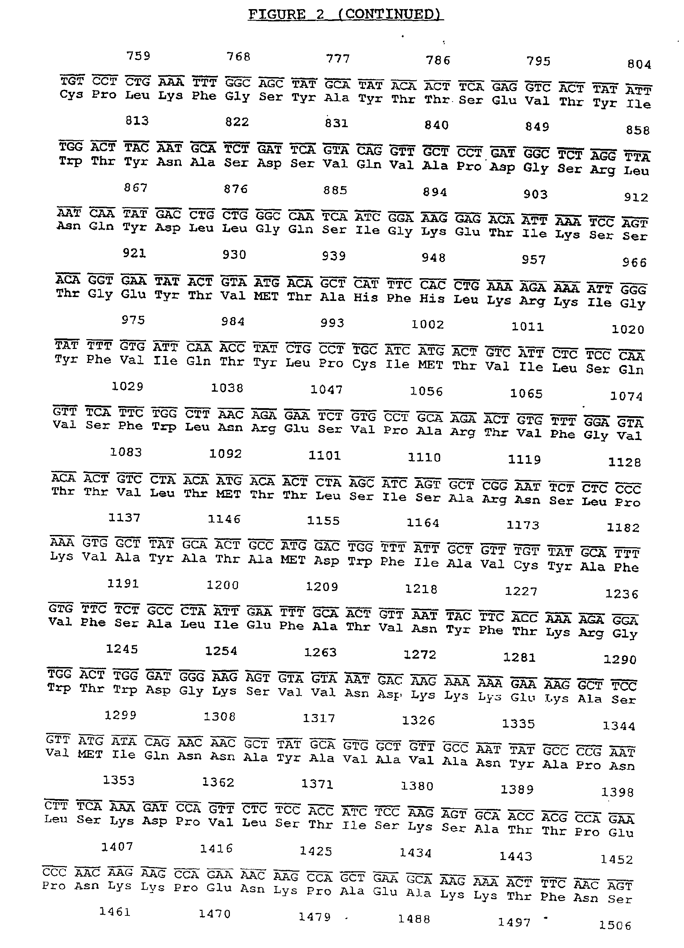

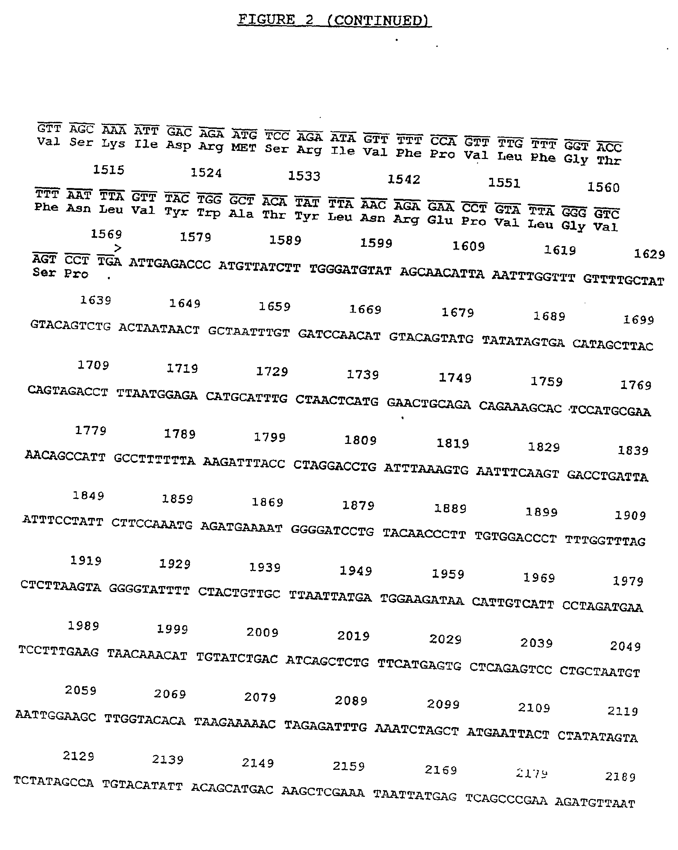

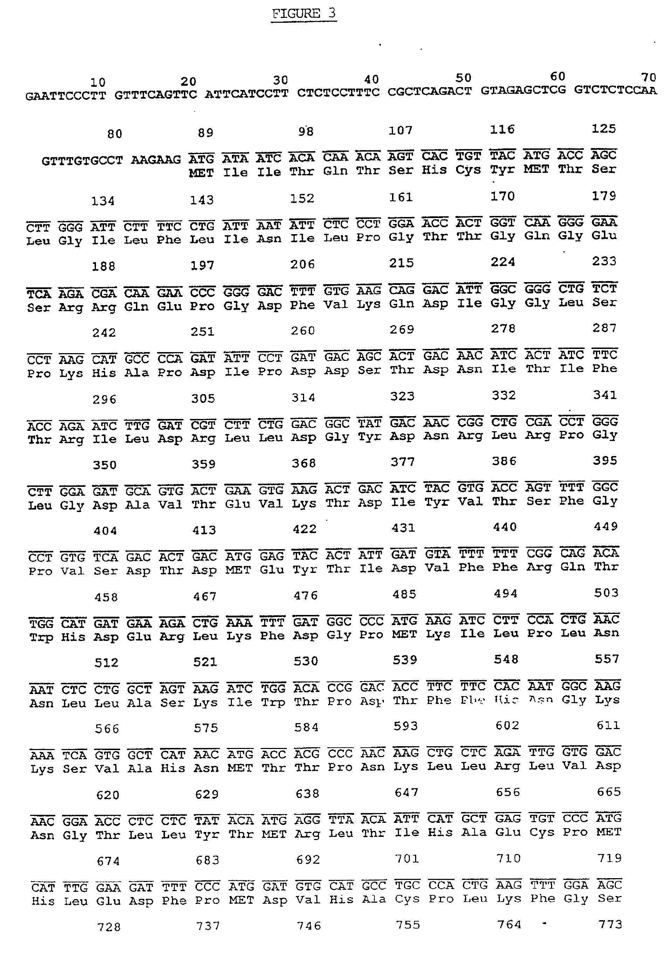

Certain cDNA sequences encoding various subunits of the human GABA_{A} receptor have hitherto been unavailable. These include in particular the sequences encoding the α₂, α₃, α₅, α₆ and β₂ subunits, which nucleotide sequences are accordingly novel. We have now ascertained the cDNA sequences of the α₂, α₃, α₅, α₆ and β₂ subunits of the human GABA_{A} receptor. These nucleotide sequences, together with the deduced amino acid sequences corresponding thereto, are depicted in Figures 2 to 6 of the accompanying drawings. The present invention accordingly provides in several additional aspects DNA molecules encoding the α₂, α₃, α₅, α₆ and β₂ subunits of the human GABA_{A} receptor comprising all or a portion of the sequences depicted in Figures 2, 3, 4, 5 and 6 respectively, or substantially similar sequences.

The sequencing of the novel cDNA molecules in accordance with the invention can conveniently be carried out by the standard procedure described in accompanying Example 3; or may be accomplished by alternative molecular cloning techniques which are well known in the art, such as those described by Maniatis et al. in Molecular Cloning, A Laboratory Manual, Cold Spring Harbor Press, New York, 2nd edition, 1989.

In another aspect, the invention provides a recombinant expression vector comprising the nucleotide sequence of a GABA_{A} receptor subunit together with additional sequences capable of directing the synthesis of the said GABA_{A} receptor subunit in cultures of stably co-transfected eukaryotic cells.

The term "expression vectors" as used herein refers to DNA sequences that are required for the transcription of cloned copies of recombinant DNA sequences or genes and the translation of their mRNAs in an appropriate host. Such vectors can be used to express eukaryotic genes in a variety of hosts such as bacteria, blue-green algae, yeast cells, insect cells, plant cells and animal cells. Specifically designed vectors allow the shuttling of DNA between bacteria-yeast, bacteria-plant or bacteria-animal cells. An appropriately constructed expression vector should contain: an origin of replication for autonomous replication in host cells, selective markers, a limited number of useful restriction enzyme sites, a high copy number, and strong promoters. A promoter is defined as a DNA sequence that directs RNA polymerase to bind to DNA and to initiate RNA synthesis. A strong promoter is one which causes mRNAs to be initiated at high frequency. Expression vectors may include, but are not limited to, cloning vectors, modified cloning vectors, specifically designed plasmids or viruses.

The term "cloning vector" as used herein refers to a DNA molecule, usually a small plasmid or bacteriophage DNA capable of self-replication in a host organism, and used to introduce a fragment of foreign DNA into a host cell. The foreign DNA combined with the vector DNA constitutes a recombinant DNA molecule which is derived from recombinant technology. Cloning vectors may include plasmids, bacteriophages, viruses and cosmids.

The recombinant expression vector in accordance with the invention may be prepared by inserting the nucleotide sequence of the chosen GABA_{A} subunit into a suitable precursor expression vector (hereinafter referred to as the "precursor vector") using conventional recombinant DNA methodology known from the art. The precursor vector may be obtained commercially, or constructed by standard techniques from known expression vectors. The precursor vector suitably contains a selection marker, typically an antibiotic resistance gene, such as the neomycin or ampicillin resistance gene. The precursor vector preferably contains a neomycin resistance gene, adjacent the SV40 early splicing and polyadenylation region; an ampicillin resistance gene; and an origin of replication, e.g. pBR322 ori. The vector also preferably contains an inducible promoter, such as MMTV-LTR (inducible with dexamethasone) or metallothionin (inducible with zinc), so that transcription can be controlled in the cell line of this invention. This reduces or avoids any problem of toxicity in the cells because of the chloride channel intrinsic to the GABA_{A} receptor.

One suitable precursor vector is pMAMneo, available from Clontech Laboratories Inc. (Lee et al., Nature, 1981, 294, 228; and Sardet et al., Cell, 1989, 56, 271). Alternatively the precursor vector pMSGneo can be constructed from the vectors pMSG and pSV2neo as described in Example 1 herein.

The recombinant expression vector of the present invention is then produced by cloning the GABA_{A} receptor subunit cDNA into the above precursor vector. The required receptor subunit cDNA is subcloned from the vector in which it is harboured, and ligated into a restriction enzyme site, e.g. the HindIII site, in the polylinker of the precursor vector, for example pMAMneo or pMSGneo, by standard cloning methodology known from the art, and in particular by techniques analogous to those described in Example 1, step (b) herein. Before this subcloning, it is often advantageous, in order to improve expression, to modify the end of a subunit cDNA with additional 5' untranslated sequences, for example by modifying the 5' end of the γ_{2L} subunit DNA by addition of 5' untranslated region sequences from the α₁ subunit DNA.

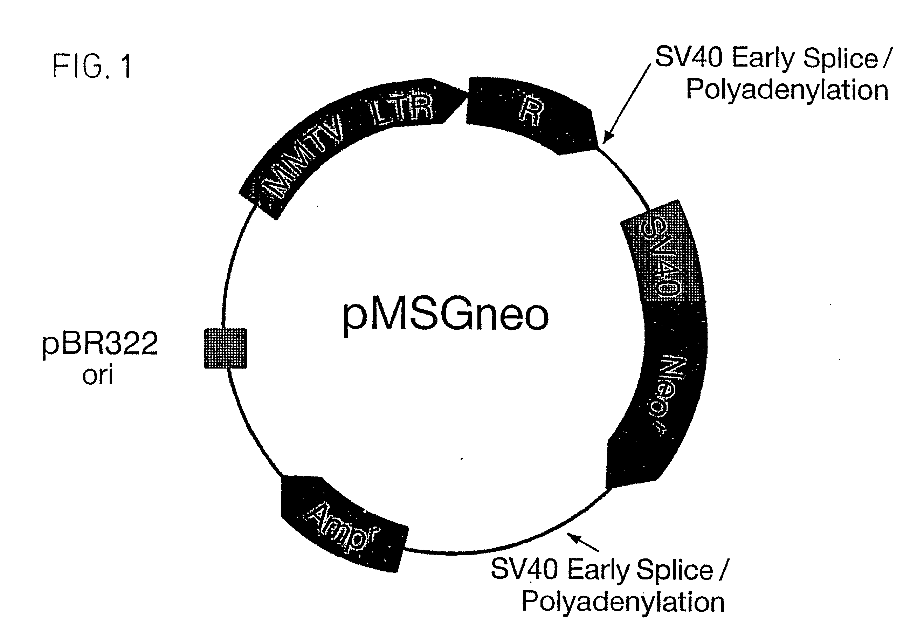

One suitable expression vector of the present invention is illustrated in Fig. 1 of the accompanying drawings, in which R represents the nucleotide sequence of a given alpha, beta or gamma subunit of the GABA_{A} receptor, and the remainder of the expression vector depicted therein is derived from the precursor vector pMSGneo and constructed as described in accompanying Example 1, steps (a) and (b).

For each cell line of the present invention, three such vectors will be necessary, one containing an α subunit, one containing a β subunit, and the third containing a γ subunit.

Cells are then co-transfected with the desired combination of three expression vectors. There are several commonly used techniques for transfection of eukaryotic cells in vitro. Calcium phosphate precipitation of DNA is most commonly used (Bachetti et al., Proc. Natl. Acad. Sci. USA, 1977, 74, 1590-1594; Maitland et al., Cell, 1977, 14, 133-141), and represents a favoured technique in the context of the present invention.

A small percentage of the host cells takes up the recombinant DNA. In a small percentage of those, the DNA will integrate into the host cell chromosome. Because the neomycin resistance gene will have been incorporated into these host cells, they can be selected by isolating the individual clones which will grow in the presence of neomycin. Each such clone is then tested to identify those which will produce the receptor. This is achieved by inducing the production, for example with dexamethasone, and then detecting the presence of receptor by means of radioligand binding..

In a further aspect, the present invention provides protein preparations of GABA_{A} receptor subunit combinations, especially human GABA_{A} receptor subunit combinations, derived from cultures of stably transfected eukaryotic cells. The invention also provides preparations of membranes containing subunit combinations of the GABA_{A} receptor, especially human GABA_{A} receptor subunit combinations, derived from cultures of stably transfected eukaryotic cells. In particular, the protein preparation and membrane preparations according to the invention will suitably contain the α₁β₁γ₂ subunit combinations of the human GABA_{A} receptor, and will preferably contain a human GABA_{A} receptor consisting of the α₁β₁γ_{2L} subunit combinations. In an especially preferred embodiment, the invention provides cell membranes containing a human GABAA receptor consisting of the α₁β₁γ_{2L} subunit combinations isolated from stably transfected mouse Ltk⁻ fibroblast cells.

The cell line, and the membrane preparations therefrom, according to the present invention have utility in screening and design of drugs which act upon the GABA_{A} receptor, for example benzodiazepines, barbiturates, β-carbolines and neurosteroids. The present invention accordingly provides the use of the cell line described above, and membrane preparations derived therefrom, in screening for and designing medicaments which act upon the GABA_{A} receptor. Of particular interest in this context are molecules capable of interacting selectively with GABA_{A} receptors made up of varying subunit combinations. As will be readily apparent, the cell line in accordance with the present invention, and the membrane preparations derived therefrom, provide ideal systems for the study of structure, pharmacology and function of the various GABA_{A} receptor subtypes.

The following non-limiting Examples illustrate the present invention.

### EXAMPLE 1

### PREPARATION OF α₁β₁γ_{2L} TRANSFECTED CELLS

### a) Construction of eukaryotic expression vector pMSGneo

The approx. 2500 base pair HindIII-EcoRI fragment of the vector pMSG (purchased from Pharmacia Biosystems Limited, Milton Keynes, United Kingdom), containing the gpt structural gene and SV40 polyadenylation signals was replaced by the approx. 2800 base pair HindIII-EcoRI fragment of pSV2neo (Southern, P.J. and Berg, P.J., Molecular and Applied Genetics, 1, 327-341, 1982) containing the neomycin resistance gene Neo^{r} and SV40 polyadenylation signals. The EcoRI and HindIII sites were then removed by restriction digesting, blunt ending with klenow polymerase, and religating. EcoRI and HindIII cloning sites were then inserted at the XhoI and SmaI sites of the polylinker by conventional techniques using EcoRI and HindIII linkers.

### b) Cloning of subunit cDNAs into pMSGneo

Bovine α₁ and β₁ GABA_{A} receptor cDNAs were obtained from the Molecular Neurobiology Unit, MRC Centre, Hills Road, Cambridge (Scholfield, P. et al. Nature, 328, 221-227, 1987). Bovine γ₂ cDNA was cloned by the method of Whiting, P. et al. (Proc. Natl. Acad. Sci. USA, 87, 9966-9970, 1990). Bovine α₁ was subcloned from pbGRαsense by digestion with EcoRI, blunt ending the DNA with klenow polymerase, addition of HindIII linkers by ligation, digestion with HindIII and ligation into the HindIII site of pMSGneo. Bovine β₁ was subcloned from pbGRβsense by restriction digestion with EcoRI (partial digestion), klenow polymerase blunt ending, ligation of HindIII linkers, restriction digestion with HindIII and ligation into HindIII site of pMSGneo. Before subcloning into pMSGneo, the bovine γ₂ cDNA was modified from the published sequence as follows. The 5' untranslated region of the bovine α₁ cDNA (bases 60-200 of the published sequence) was added to the 5' end of the published γ₂ sequence by amplifying the α₁ untranslated region using polymerase chain reaction, and then subcloning the product into the 5' BamHI (site in the polylinker of the Bluescript Sk⁻ cloning vector; Bluescript vector purchased from Stratagene, San Diego, U.S.A.) HindIII sites of the γ₂ cDNA. The modified γ₂ cDNA was then subcloned into pMSGneo by digestion with XbaI (site in the polylinker of the cloning vector), blunt ending with klenow polymerase, ligation of XhoI linkers, digestion with XhoI (site in the polylinker of the cloning vector), and ligation into XhoI site of pMSGneo.

### c) Co-transfection of mouse Ltk⁻ cells

Ltk⁻ cells were obtained from the Salk Institute for Biological Studies, San Diego, California. Cells were grown at 37°C, 5-8% CO₂, in Modified Eagles Medium containing penicillin, streptomycin and 10% fetal calf serum. The expression vector harbouring the GABA_{A} receptor subunit DNAs for co-transfection was prepared by a standard protocol (Chen, C. and Okayama, H., BioTechniques, 6, 632-638, 1988). For co-transfection, Ltk⁻ cells were plated in dishes (approx. 2x10⁵ cells/dish) and grown overnight. The transfection was performed by calcium phosphate precipitation using a kit (purchased from 5 Prime -> 3 Prime Products, Westchester, Pennsylvania). Co-transfection was performed according to manufacturers' instructions, using 5µg of each subunit DNA construct per 10cm dish of cells. After 2 days in culture the cells were divided 1:8 into culture medium containing 1mg/ml neomycin [Geneticin (obtainable from Gibco BRL, Paisley, Scotland, U.K.)]. After a further week the concentration was increased to 1.5mg/ml, and then 2mg/ml 1 week after that. Resistant clones of cells were isolated and subcloned using cloning cylinders. Subclones were analysed using radioligand binding: subclones were grown in 10cm culture dishes, and when confluent changed into culture medium containing 1µM dexamethasone (obtainable from Sigma Chemical Company, Poole, Dorset, United Kingdom). 3-5 days later the cells were harvested, membranes prepared and used for radioligand binding (see Example 2, step (a) below) using the benzodiazepine antagonist ³H Ro15-178 (obtained from New England Nuclear, Du Pont (U.K.) Ltd, Stevenage, United Kingdom). The clone expressing the highest amount of ³H Ro15-1788 binding was subcloned from a single cell by limiting dilution. The resultant clonal population of cells described below is referred to as population A.

### EXAMPLE 2

### CHARACTERIZATION OF α₁β₁γ_{2L} TRANSFECTED CELLS

### a) Radioligand binding

The nature of the recombinant α₁β₁γ_{2L} GABA_{A} receptors prepared as described in Example 1 was addressed by characterization of the benzodiazepine (BZ) binding pharmacology, using the BZ antagonist ³H Ro15-1788. For radioligand binding assays, cells which had been induced by culture in dexamethasone containing medium for 3-5 days were scraped off into 50mM Tris, pH7.5, 100mM NaCl in the form of Tris buffered saline (TBS) and pelleted (20,000rpm, Sorvall RC5C centrifuge). The cell pellet was resuspended in 50mM Tris, pH7.5, homogenised using an Ultra-Turrax homogeniser and then pelleted as above. This was repeated once more, and the cells then resuspended in TBS (0.4ml per original 10cm dish of cells). Radioligand binding was performed in 0.1ml final volume TBS, containing 5-15 fmols of ³H Ro15-1788 binding sites. After 1 hour incubation on ice the membranes were harvested onto filters using a Brandel cell harvester, washed with cold TBS, and bound radioactivity determined by scintillation counting. The recombinant α₁β₁γ_{2L} receptors bound ³H Ro15-1788 with high affinity (K_{D} 0.4nM), at levels of up to 200fmols/10cm dish of cells. No binding was seen to either untransfected Ltk⁻ cells, or population A cells which had not been induced by addition of dexamethasone to the culture medium, confirming that the ³H Ro15-1788 was binding to recombinant α₁β₁γ₂ GABA_{A} receptors. The ³H Ro15-1788 binding was inhibited by flunitrazepam, CL218872, FG8205, βCCM, zolpidem and Ro15-4513, confirming the BZ pharmacology of the recombinant receptor. Since it is established that only GABA_{A} receptors containing an α, a β and a γ subunit exhibit BZ binding (Pritchett, D. et al., Nature, 338, 582-585, 1989) these data confirm the nature of the recombinant α₁β₁γ₂ GABA_{A} receptors expressed by population A cells.

### b) Electrophysiology

The nature of the GABA_{A} receptor expressed by population A cells has been extensively characterised by electrophysiological techniques, using whole cell patch clamp. Only cells induced by culture in the presence of dexamethasone showed responses to GABA. Concentration response curves to GABA gave a log EC₅₀ of 5.2, and a Hill coefficient of 1.9. The response to GABA was potentiated by BZs flunitrazepam and CL218872, by the barbiturate pentobarbitone, and by the steroid alphaxalone. The response to GABA was antagonised by both bicuculline and picrotoxin. All these electrophysiological data confirm that the recombinant GABA_{A} receptor expressed by population A cells has all of the properties expected of a bona fide GABA_{A} receptor.

### EXAMPLE 3

### ISOLATION AND SEQUENCING OF cDNAS ENCODING HUMAN GABA_{A} RECEPTOR α₂, α₃, α₅, α₆ & β₂ SUBUNITS

### a) cDNA libraries

cDNAs were cloned from human foetal brain (α₂, α₃), hippocampal (α₅, β₂) and cerebellum (α₆) lambda bacteriophage cDNA libraries. All cDNA libraries were constructed in the lambdaZAP vector, and were purchased from Stratagene (San Diego, California). For screening, the cDNA libraries were plated according to the manufacturer's instructions, at 40,000 pfu per 137 mm plate. Filter lifts were taken using Hybond N filters (Amersham) according to the manufacturer's instructions.

### b) Isolation of cDNA encoding human α₂ subunit

A bovine α₂ cDNA (obtained from E. Barnard, Molecular Neurobiology, University of Cambridge, Hills Road, Cambridge; Levitan et al., Nature, 1988, 335, 76) was labelled to high specific activity (>1.10⁹ cpm/µg) with ³²P by random priming and used as a probe. Library filters (8 replica filters) were prehybridised for 3-6 hours at 42°C in 5x SSPE (1x SSPE is 0.18M NaCl, 0.01M Na₃PO₄ [pH7.4], 1mM EDTA), 5x Denhardt's solution, 100 µg/ml salmon sperm DNA, 0.1% sodium dodecyl sulphate (SDS), 30% formamide. Hybridisation was performed in the same buffer for 18 hours at 42°C, including 0.5-1.10⁶ cpm ³²P-labelled probe per ml of hybridisation buffer. Filters were washed at 55°C in 5x SSPE (2x 15 minutes) and 1x SSPE (2x 15 minutes) and exposed to Kodak XAR film for 1-3 days. Positive clones were plaque purified using standard techniques, and the Bluescript plasmid (Stratagene) "rescued" according to manufacturer's instructions. cDNA clones were sequenced on both strands by standard techniques using Sequenase II enzyme (United States Biochemicals). The nucleotide sequence of the cDNA encoding the human GABA_{A} receptor α₂ subunit, together with the deduced amino acid sequence corresponding thereto, is shown in Fig. 2 of the accompanying drawings.

### c) Isolation of cDNA encoding human α₃ subunit

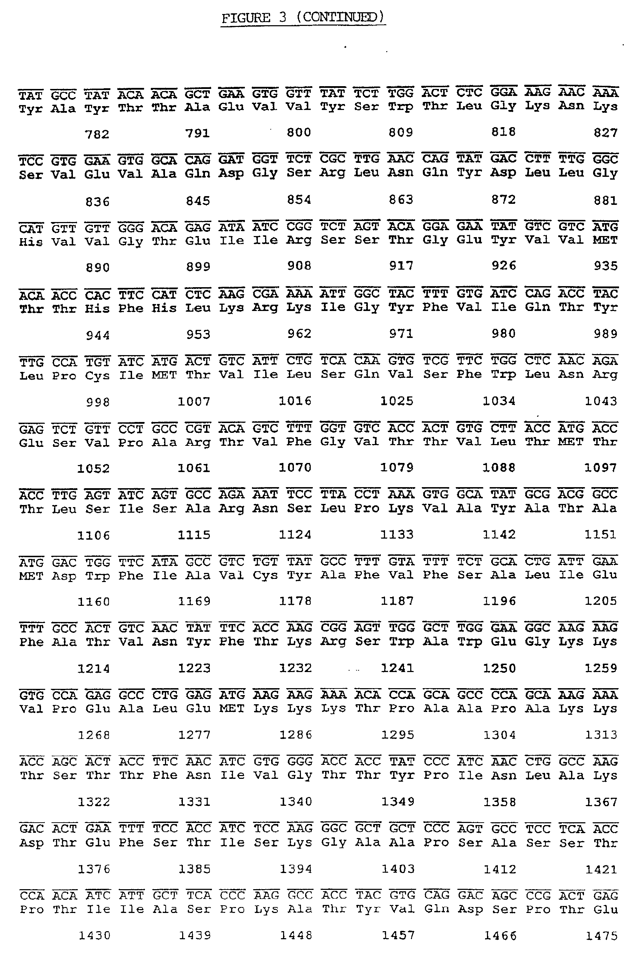

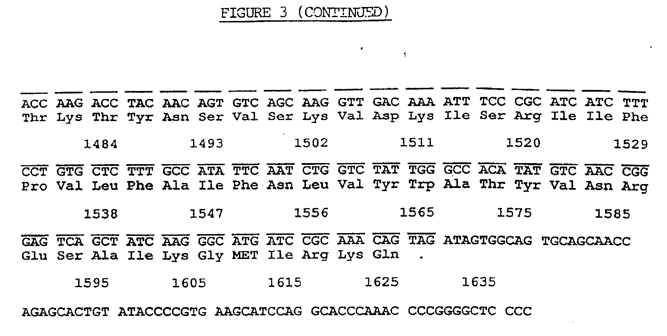

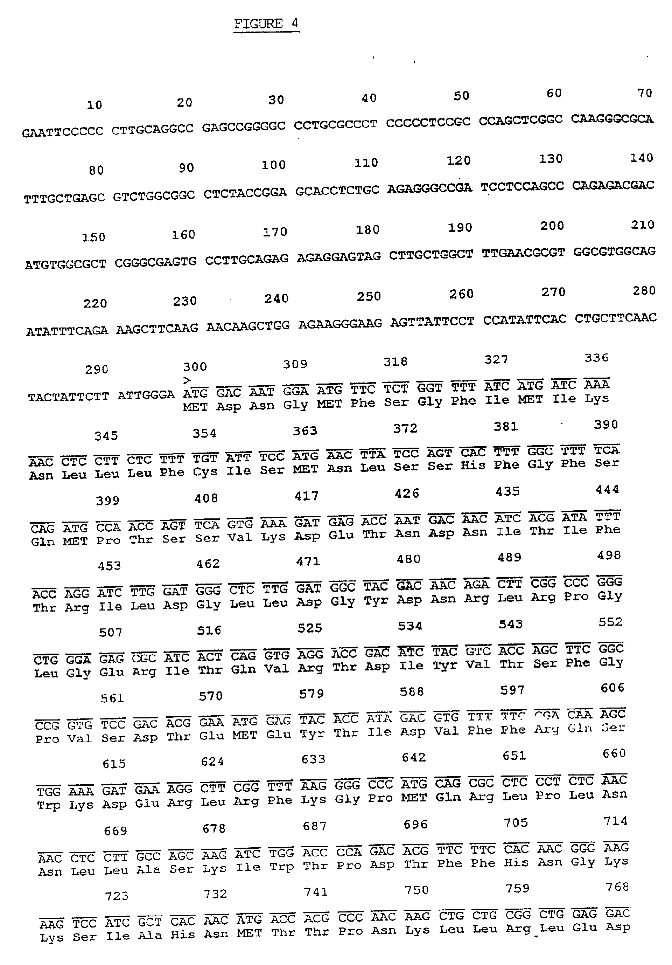

A bovine α₃ cDNA (obtained from E. Barnard, Molecular Neurobiology, University of Cambridge, Hills Road, Cambridge; Levitan et al., Nature, 1988, 335, 76) was labelled to high specific activity with ³²P by random priming and used as a probe. Library filters were prehybridised for 3-6 hours at 55°C in 5x SSPE, 5x Denhardt's solution, 0.1% SDS, 100 µg/ml salmon sperm DNA, and hybridised for 18 hours, 55°C in the same buffer, containing 0.5-1x 10⁶ cpm/ml of ³²P-labelled bovine α₃ cDNA as probe. Filters were washed and exposed to X-ray film as described above; cDNA clones were rescued and sequenced as described above. The longest α₃ cDNA clone was missing in approximately 100 bp of the 5' end of the coding region. This was obtained by PCR using as primers an oligonucleotide "anchor" primer derived from the T7 primer sequence of Bluescript vector (5'AGCGCGCGTAATACGACTCACTATAGGGCGAA3') and an oligonucleotide derived from sequence near the 5' end of the truncated α₃ cDNA, containing an internal Hpal site (5'CAGCATGAATTGTTAACCTCATTGTA3'). Oligonucleotides were synthesised on an Applied Biosystems 380B synthesiser. PCR was performed as described above, and a 300bp PCR product obtained which was double digested with Hpal and Kpnl and subcloned into the similarly cut truncated α₃ cDNA to yield a full length human α₃ cDNA. The cDNA was sequenced on both strands as described above. The nucleotide sequence of the cDNA encoding the human GABA_{A} receptor α₃ subunit, together with the deduced amino acid sequence corresponding thereto, is shown in Fig. 3 of the accompanying drawings.

### d) Isolation of cDNA encoding human α₅ subunit

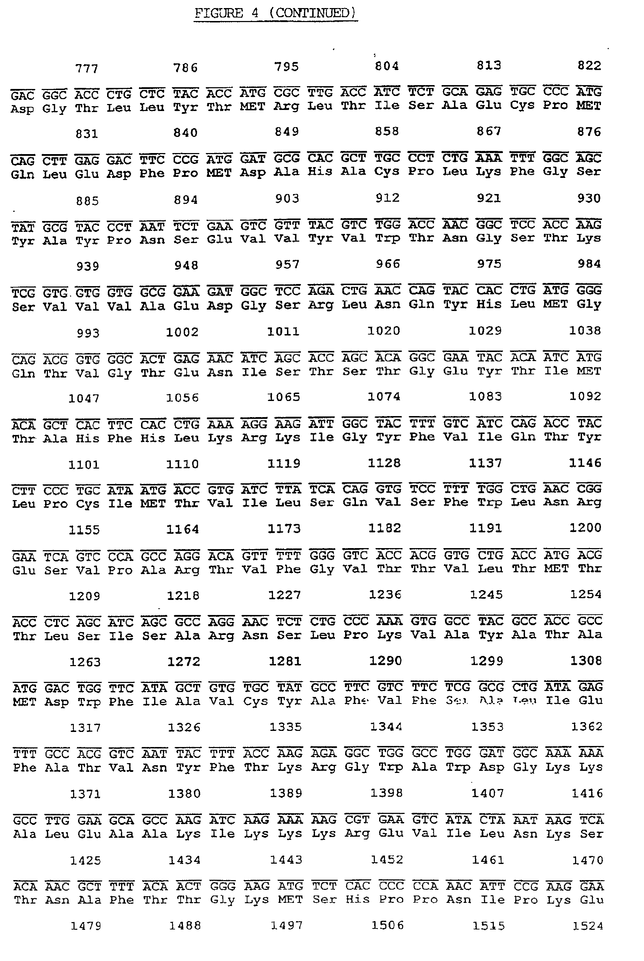

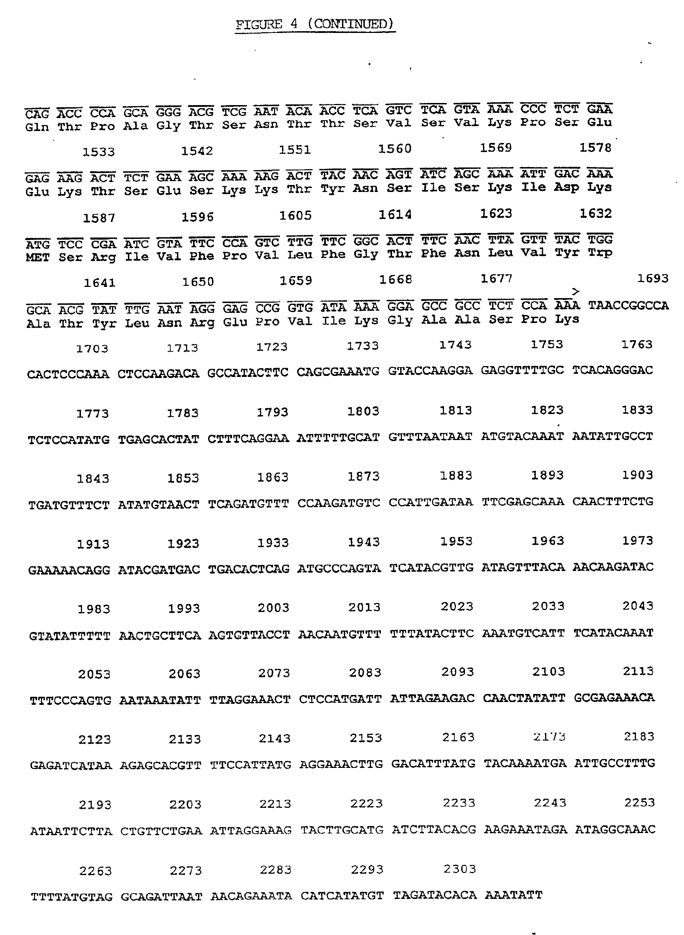

A rat α₅ cDNA obtained by polymerase chain reaction (PCR) was used as a probe to screen the cDNA library. For PCR, sequences of the oligonucleotide primers were taken from the published α₅ sequences (Khrestchatisky et al., Neuron, 1989, 3, 745) and incorporated a Hind III site for subcloning purposes: 5' ATTATTCAAGCTTGCCATGGACAATGGAATGCTC3' (bp114-148); 5'GGTTTCCAGCTTACTTTGGAGAGGTAGC3' (bp1507-1535). PCR and subcloning of the PCR product into Bluescript SK-vector (Stratagene) for analysis was performed as described elsewhere (Whiting et al., Proc. Natl. Acad. Sci. USA, 1990, 87, 9966) except that rat brain cDNA was used as template. The rat α₅ cDNA was labelled with ³²P and used to screen the human hippocampal cDNA library, and positive α₅ clones rescued and sequenced as described for α₂ above. The nucleotide sequence of the cDNA encoding the human GABA_{A} receptor α₅ subunit, together with the deduced amino acid sequence corresponding thereto, is shown in Fig. 4 of the accompanying drawings.

### e) Isolation of cDNA encoding human α₆ subunit

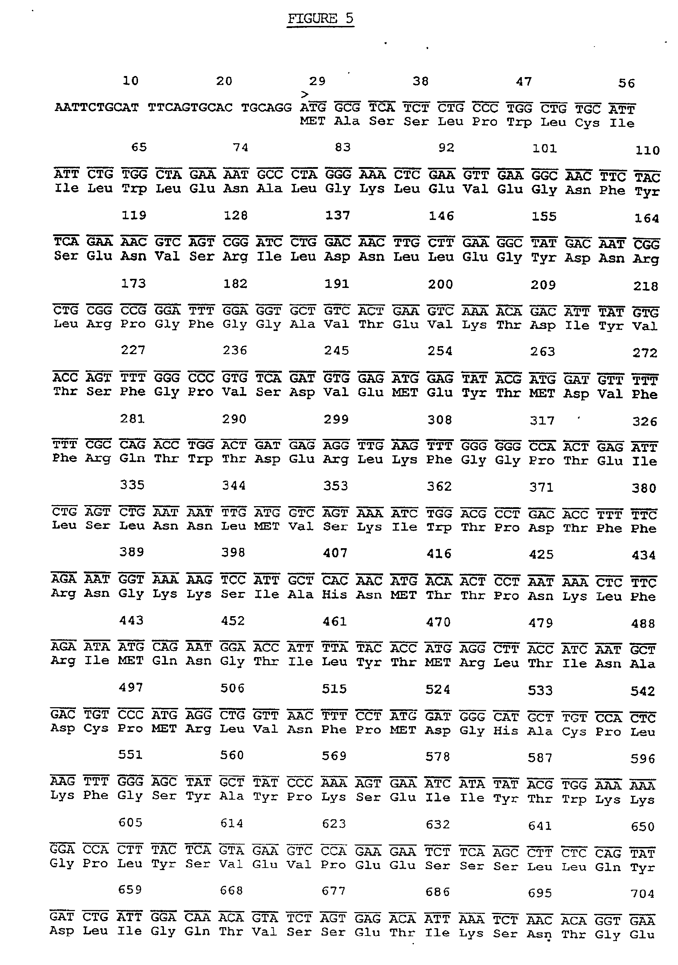

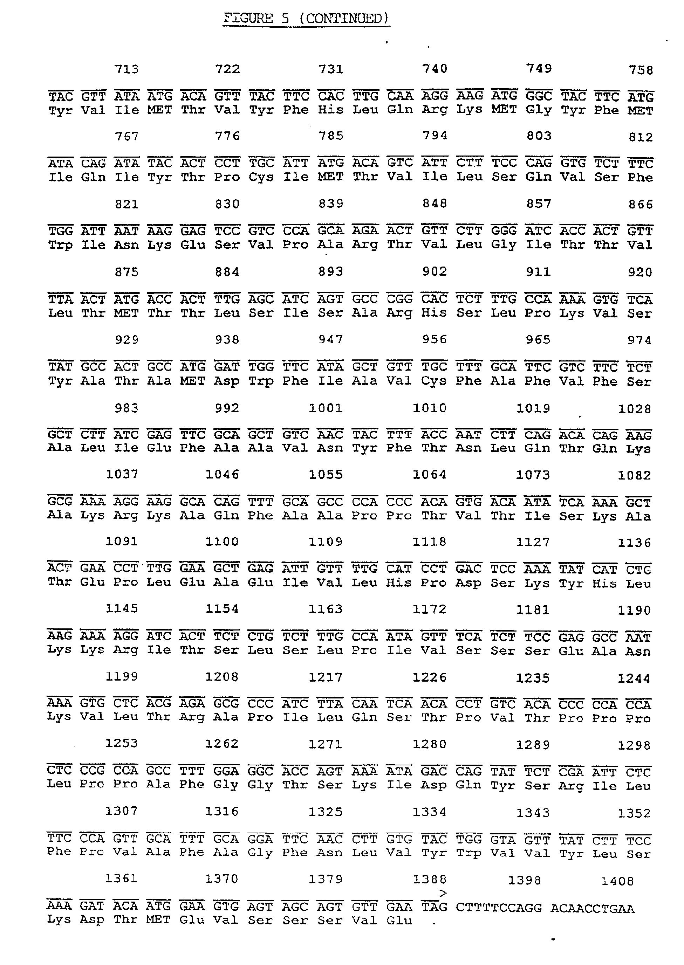

A rat α₆ cDNA obtained by PCR was used as a probe to screen the cDNA library. PCR was performed as described above for α₅, using oligonucleotide primers derived from the published rat α₆ sequence (Luddens et al., Nature, 1990, 346, 648) incorporating an EcoRI site for subcloning purposes: 5'GAGGAAGAATTCAGGAGGGTGACCT3' (bp48-72); 5'GAAAATAACGAATTCCAGTGTCCAGCTTT3' (bp1376-1404). The rat α₆ cDNA clone isolated by PCR was labelled with ³²P and used to screen a human cerebellum cDNA library, as described above for α₂. Positive α₆ clones were purified, rescued and sequenced as described above. None of the cDNAs contained a complete coding region. To obtain a full length cDNA 3 clones were joined together using convenient restriction sites. The nucleotide sequence of the cDNA encoding the human GABA_{A} receptor α₆ subunit, together with the deduced amino acid sequence corresponding thereto, is shown in Fig. 5 of the accompanying drawings.

### f) Isolation of cDNA encoding human β₂ subunit

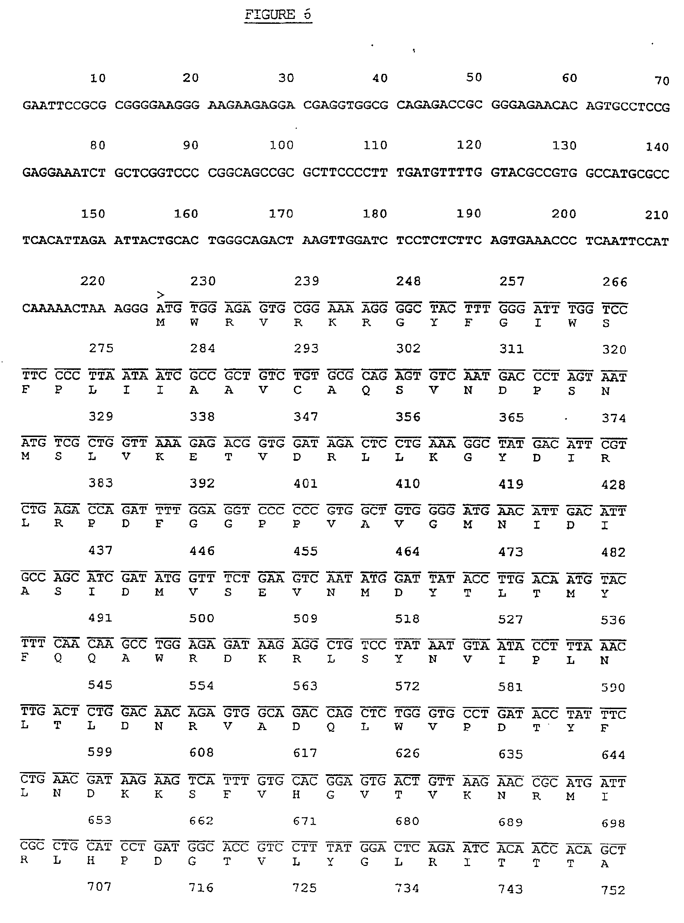

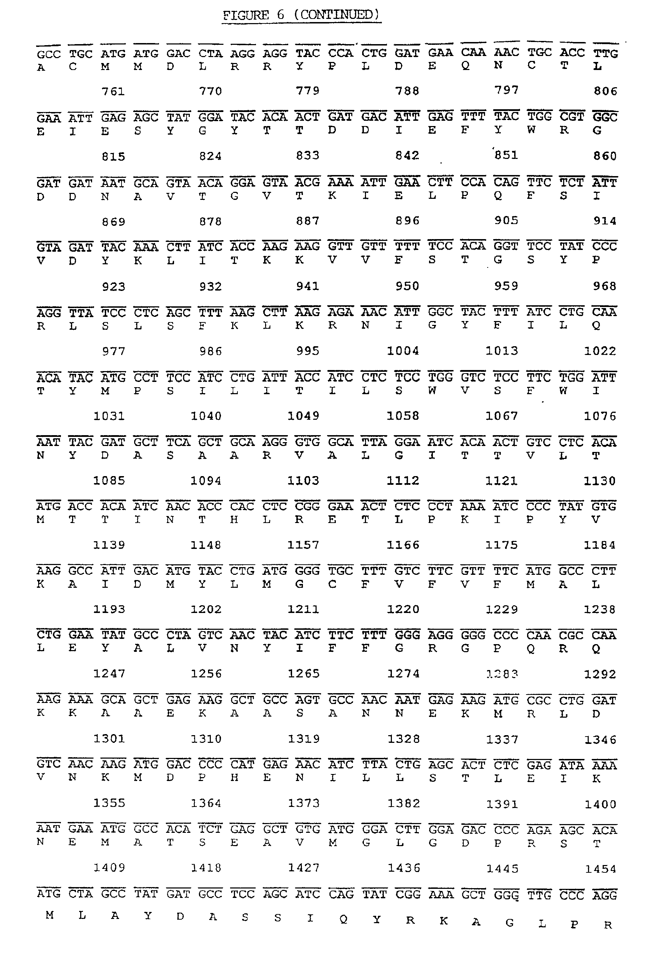

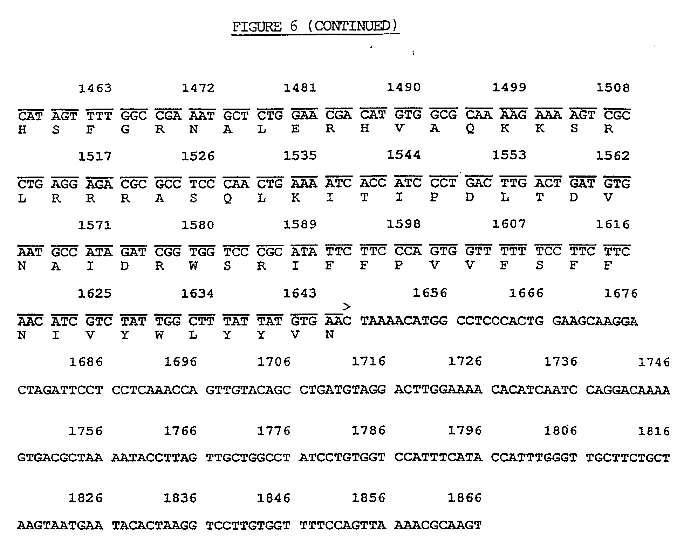

Human β₂ cDNA was isolated using as a probe a short human β₂ cDNA obtained by PCR. PCR was performed as described above (except that the human cerebellum cDNA library was used as template), using oligonucleotide primers derived from the published rat β₂ sequence (Ymer et al., EMBO J., 1989, 8, 1665), incorporating EcoRI sites for subcloning purposes: 5' CAAAAGAATTCAGCTGAGAAAGCTGCTAATGC3' (bp1088-1119); 5' TCAGGCGAATTCTCTTTTGTGCCACATGTCGTTC3' (bp1331-1364). The human β₂ clone obtained by PCR was radiolabelled with ³²P and used to screen a human hippocampal cDNA library, as described above for α₂. The largest cDNA clone obtained lacked the 5' 500bp of the coding region of the β₂ subunit. This was obtained by PCR using as primers an oligonucleotide "anchor" primer derived from the T7 primer sequence of the Bluescript vector (5' AGCGCGCGTAATACGACTCACTATAGGGCGAA3'), and an oligonucleotide derived from sequence near the 5' end of the truncated β₂ cDNA, containing a Kpnl site (5' CATCCAGTGGGTACCTCCTTAGGT3'). PCR was performed as described above, and a 700bp PCR product obtained which was digested with kpnl and subcloned into the truncated cDNA clone (also Kpnl digested) to yield a full length human β₂ cDNA. The nucleotide sequence of the cDNA encoding the human GABA_{A} receptor β₂ subunit, together with the deduced amino acid sequence corresponding thereto, is shown in Fig. 6 of the accompanying drawings.

## Claims

1. A stably co-transfected eukaryotic cell line capable of expressing a GABA_{A} receptor, which receptor comprises at least one alpha, at least one beta and at least one gamma subunit.

2. A cell line as claimed in claim 1 wherein the receptor expressed is a human GABA_{A} receptor.

3. A process for the preparation of a eukaryotic cell line capable of expressing a GABA_{A} receptor, which comprises stably co-transfecting a eukaryotic host cell with at least three expression vectors, one such vector harbouring the cDNA sequence encoding for an alpha, another such vector harbouring the cDNA sequence for a beta, and a third such vector harbouring the cDNA sequence encoding for a gamma GABA_{A} receptor subunit.

4. A process as claimed in claim 3 wherein the eukaryotic host cell employed is a rodent fibroblast cell.

5. A DNA molecule encoding the α₂ subunit of the human GABA_{A} receptor comprising all or a portion of the sequence depicted in Figure 2 herein, or a substantially similar sequence.

6. A DNA molecule encoding the α₃ subunit of the human GABA_{A} receptor comprising all of a portion of the sequence depicted in Figure 3 herein, or a substantially similar sequence.

7. A DNA molecule encoding the α₅ subunit of the human GABA_{A} receptor comprising all or a portion of the sequence depicted in Figure 4 herein, or a substantially similar sequence.

8. A DNA molecule encoding the α₆ subunit of the human GABA_{A} receptor comprising all or a portion of the sequence depicted in Figure 5 herein, or a substantially similar sequence.

9. A DNA molecule encoding the β₂ subunit of the human GABA_{A} receptor comprising all or a portion of the sequence depicted in Figure 6 herein, or a substantially similar sequence.

10. A recombinant expression vector comprising the nucleotide sequence of a GABA_{A} receptor subunit together with additional sequences capable of directing the synthesis of the said GABA_{A} receptor subunit in cultures of stably co-transfected eukaryotic cells.

11. A protein preparation of GABA_{A} receptor subunit combinations derived from a culture of stably co-transfected eukaryotic cells.

12. A membrane preparation containing subunit combinations of the GABA_{A} receptor derived from a culture of stably co-transfected eukaryotic cells.

13. A preparation as claimed in claim 11 or claim 12 wherein the subunit combinations derived are human GABA_{A} receptor subunit combinations.

14. A preparation as claimed in claim 13 wherein the subunit combination derived is the α₁β₁γ₂ subunit combination of the human GABA_{A} receptor.

15. A preparation as claimed in claim 14 containing a human GABA_{A} receptor consisting of the α₁β₁γ_{2L} subunit combination isolated from stably transfected mouse Ltk⁻ fibroblast cells.

16. The use of the cell line as claimed in claim 1 or claim 2, and membrane preparations derived therefrom, in screening for and designing medicaments which act upon the GABA_{A} receptor.
